# EUROPÄISCHE PATENTSCHRIFT

(11) **EP 2 851 102 B1**
(45) Veröffentlichungstag und Bekanntmachung des Hinweises auf die Patenterteilung: **30.10.2019**
(21) Anmeldenummer: 14184113.0
(22) Anmeldetag: 09.09.2014
(51) Int. Cl.: A61M 27/00, A61M 1/00

(54) **Medizinisches Kit zur Ableitung von pathologischen Fluidansammlungen**
Medical Kit for the draining pathological fluids.
Kit médical pour drainer des fluides pathologiques

(30) Priorität: 13.09.2013 DE 102013218407
(43) Veröffentlichungstag der Anmeldung: 25.03.2015
(73) Patentinhaber: AESCULAP AG, 78532 Tuttlingen (DE)
(72) Erfinder: Abele, Wolfgang, 78532 Tuttlingen (DE); Odermatt, Erich, 8200 Schaffhausen (CH)
(74) Vertreter: Patentanwälte Ruff, Wilhelm, Beier, Dauster & Partner mbB

(56) Entgegenhaltungen:
- EP-A1- 2 769 744
- WO-A1-89/11308
- WO-A1-2011/038949
- WO-A2-03/028786
- DE-A1-102008 061 536

## Beschreibung

Die Erfindung betrifft ein medizinisches Kit zur Ableitung von pathologischen Fluidansammlungen, einen Fluidkommunikationskörper sowie ein Führungselement.

Aus der EP 2 769 744 A1 ist ein medizinisches Produkt zur Ableitung von pathologischen Fluidansammlungen bekannt, welches einen Fluidsammelkörper, einen Fluidkommunikationskörper sowie ein Zugelement zur Platzierung des Produkts in einen menschlichen oder tierischen Körper umfasst.

Die WO 89/11308 A1 offenbart ein intranasal zu applizierendes Produkt, welches neben einem Absorptionskörper, einem Inflationsschlauch, einem Drainageschlauch und einer expandierbaren Manschette ein Führungsende aufweist.

Eine Behandlungsmöglichkeit von infektiösen Körperhöhlen stellt die sogenannte endoluminale und endokavitäre Vakuumtherapie dar. Bei dieser Therapieform kommt ein spezielles Drainagesystem zum Einsatz, welches sich im Wesentlichen aus einem offenporösen und insbesondere schwammförmigen Fluidsammelkörper und einem schlauchförmigen Fluidkommunikationskörper, der mit dem Fluidsammelkörper verbunden ist, zusammensetzt. Der Fluidsammelkörper dient zum Aufsaugen von Wundsekreten, während der Fluidkommunikationskörper für die Abführung bzw. Ableitung der aufgesogenen Wundsekrete verantwortlich ist. Ein solches Drainagesystem wird beispielsweise von der Anmelderin unter der Bezeichnung Endo-Sponge® kommerziell vertrieben.

In der Regel wird der Fluidsammelkörper zusammen mit dem Fluidkommunikationskörper mit Hilfe eines Überschlauches (Overtube) und unter endoskopischer Kontrolle in eine menschliche oder tierische Körperhöhle platziert.

Um eine erfolgreiche Therapie zu gewährleisten, muss der Fluidsammelkörper in der Regel nach etwa drei Tagen ausgetauscht werden.

Diese Vorgehensweise ist mit diversen Nachteilen behaftet. So kann das umliegende Gewebe in den Fluidsammelkörper einwachsen, ehe der Fluidsammelkörper ausgetauscht wird. Dies wiederum kann zur Folge haben, dass der Fluidsammelkörper während seiner Entfernung aus einer Körperhöhle fragmentiert und die Entfernung als solche für den Patienten schmerzhaft ist.

Ein weiterer Nachteil besteht darin, dass der Fluidsammelkörper zusammen mit dem Fluidkommunikationskörper entfernt werden muss, so dass im Ergebnis nicht nur ein neuer Fluidsammelkörper, sondern auch ein neuer Fluidkommunikationskörper positioniert werden muss. Dies macht eine erneute endoskopische Kontrolle erforderlich, wodurch das Behandlungsverfahren verkompliziert wird.

### Aufgabe und Lösung

Es ist daher eine Aufgabe der Erfindung, ein medizinisches Kit bereitzustellen, welches eine einfachere, flexiblere und insbesondere unter Behandlungsgesichtspunkten manipulativere Ableitung von pathologischen Fluidansammlungen und insbesondere eine zerstörungsfreie Entfernung eines Fluidsammelkörpers aus einer menschlichen oder tierischen Körperhöhle erlaubt. Dies wird erfindungsgemäß durch ein medizinisches Kit gemäß Anspruch 1 erreicht. Bevorzugte Ausführungsformen des Kits sind in den Unteransprüchen 2 bis 12 definiert. Der Wortlaut sämtlicher Ansprüche wird hiermit durch ausdrückliche Bezugnahme zum Inhalt der vorliegenden Beschreibung gemacht. Verweise auf Ausführungsformen in der Beschreibung, die nicht unter den Anwendungsbereich der beigefügten Ansprüche fallen, stellen lediglich mögliche exemplarische Ausführungen dar und sind daher nicht Teil der vorliegenden Erfindung

Die Erfindung betrifft gemäß einem ersten Aspekt ein medizinisches Kit zur Ableitung von pathologischen Fluidansammlungen, vorzugsweise zur Durchführung einer endoluminalen und/oder -kavitären Vakuumtherapie.

Erfindungsgemäß umfasst das Kit wenigstens die Kitkomponenten
a) einen Fluidsammelkörper und einen Fluidkommunikationskörper,
b) einen Fluidsammelkörper und eine Sicherungseinrichtung oder
c) einen Fluidkommunikationskörper und eine Sicherungseinrichtung.

Das Kit umfasst einen Fluidsammelkörper, einen Fluidkommunikationskörper und eine Sicherungseinrichtung. Der Fluidsammelkörper und der Fluidkommunikationskörper liegen vorzugsweise räumlich voneinander getrennt vor.

Der Fluidsammelkörper und der Fluidkommunikationskörper sind koaxial zueinander und verschieblich gegeneinander anordenbar.

Weiterhin sind der Fluidsammelkörper und der Fluidkommunikationskörper derart anordenbar, dass der Fluidsammelkörper den Fluidkommunikationskörper teilweise, insbesondere nur teilweise, umgibt.

Die Sicherungseinrichtung ist dazu ausgebildet, den Fluidsammelkörper gegen ein Durchschieben über ein distales Ende des Fluidkommunikationskörpers hinaus zu sichern. Abhängig von der Ausgestaltung der Sicherungseinrichtung kann diese obendrein zur Verankerung bzw. Befestigung und/oder Positionierung des Fluidsammelkörpers und/oder des Fluidkommunikationskörpers innerhalb einer Körperhöhle vorgesehen sein und insoweit zusätzlich als Verankerungs- bzw. Befestigungs- und/oder Positionierungseinrichtung wirken.

Durch die koaxiale Anordenbarkeit/Anordnung von Fluidsammelkörper und Fluidkommunikationskörper, die gegenseitige Verschieblichkeit/Verschiebbarkeit von Fluidsammelkörper und Fluidkommunikationskörper sowie insbesondere durch die Anwesenheit einer Sicherungseinrichtung, die gegebenenfalls zusätzlich als Verankerungs- bzw. Befestigungs- und/oder Positionierungseinrichtung wirken kann, ergeben sich insgesamt mehr Möglichkeiten, um die Ableitung von pathologischen Fluidansammlungen, insbesondere im Rahmen einer endoluminalen und/oder -kavitären Vakuumtherapie, zu vereinfachen und vor allem in Richtung eines Behandlungserfolges günstig zu beeinflussen:
- So entfällt aufgrund der besonderen Ausbildung (gegenseitige Verschieblichkeit/Verschiebbarkeit) von Fluidsammelkörper und Fluidkommunikationskörper eine gegenseitige Verbindung, insbesondere eine gegenseitige Verklebung und/oder ein gegenseitiges Vernähen, von Fluidsammelkörper und Fluidkommunikationskörper.
   Dies vereinfacht nicht nur die Ableitung von Fluidansammlungen, sondern ermöglicht allgemein auch einen flexibleren Einsatz von Fluidsammelkörper und Fluidkommunikationskörper.
- Weiterhin ist vorteilhaft, dass zur Ableitung von Fluidansammlungen aus einer menschlichen oder tierischen Körperhöhle lediglich der Fluidkommunikationskörper (und ein zusätzlich vorgesehenes Führungselement) mittels eines Endoskops positioniert werden müssen. Im Falle des anschließend auf den Fluidkommunikationskörper aufzubringenden Fluidsammelkörpers ist eine endoskopische Kontrolle nicht erforderlich, da der positionierte Fluidkommunikationskörper als solcher die Funktion eines Führungselementes übernehmen kann. Dies kann beispielsweise durch einfaches Aufschieben des Fluidsammelkörpers auf den Fluidkommunikationskörper geschehen.
- Ein weiterer Vorteil besteht darin, dass nach einer bestimmten Behandlungszeit lediglich ein Austausch des Fluidsammelkörpers, indes nicht des Fluidkommunikationskörpers erforderlich ist. Letzterer kann somit in der Körperhöhle verbleiben, beispielsweise gesichert durch eine Sicherungseinrichtung, die zusätzlich als Verankerungseinrichtung wirkt.
- Weiterhin von Vorteil ist, dass vor dem Aufbringen des Fluidsammelkörpers auf den Fluidkommunikationskörper ein patientenspezifischer Zuschnitt des Fluidsammelkörpers auch noch unmittelbar vor dessen Positionierung in eine menschliche oder tierische Körperhöhle erfolgen kann.
- Durch die Anwesenheit einer Sicherungseinrichtung am distalen Ende des Fluidkommunikationskörpers kann verhindert werden, dass der Fluidsammelkörper unbeabsichtigt über das distale Ende des Fluidkommunikationskörpers hinaus geschoben wird. Eine gegenseitige Verbindung, insbesondere eine gegenseitige Verklebung und/oder ein gegenseitiges Vernähen, von Fluidsammelkörper und Fluidkommunikationskörper, wie bei gattungsgemäßen Drainagesystemen, ist somit zur Verhinderung eines ungewollten Durchschiebens des Fluidsammelkörpers über das distale Ende des Fluidkommunikationskörpers hinaus nicht erforderlich.
   Die Anwesenheit einer Sicherungseinrichtung am distalen Ende des Fluidkommunikationskörpers erleichtert zudem die Entfernung eines positionierten Fluidsammelkörpers. So kann der Fluidsammelkörper bei Zugbeanspruchung des Fluidkommunikationskörpers mittels der Sicherungseinrichtung an seinem distalen Ende komprimiert und schonend aus der Körperhöhle entfernt werden. Durch wiederholtes Vor- und Zurückziehen des Fluidkommunikationskörpers lässt sich der Fluidsammelkörper in Etappen und damit auf eine für den Patienten behutsame und (wenig) atraumatische Weise aus einer Körperhöhle entfernen. Insgesamt lässt sich der Fluidsammelkörper dadurch besser von einer Körperhöhlenwandung ablösen, was insbesondere bei Vorliegen von Gewebeverwachsungen von Vorteil ist.

Alternativ kann der Fluidsammelkörper auch regelmäßig zugbeansprucht oder an seinem distalen Ende, beispielsweise durch Überstülpen eines Rohres, komprimiert werden, um Gewebeverwachsungen im Voraus vorzubeugen.

Unter dem Ausdruck "Fluidsammelkörper" soll im Sinne der vorliegenden Erfindung ein Körper verstanden werden, der dazu befähigt ist, pathologische Fluidansammlungen aufzunehmen, bevorzugt aufzusaugen, und insbesondere zu sammeln sowie die aufgenommenen und insbesondere gesammelten Fluide, in der Regel durch Anlegen eines Unterdrucks oder Vakuums, an einen Fluidkommunikationskörper abzugeben.

Unter dem Begriff "Fluidkommunikationskörper" soll im Sinne der vorliegenden Erfindung ein Körper verstanden werden, der in der Lage ist, eine Fluidkommunikation zwischen einem Fluidsammelkörper und einem Fluidauffangkörper, wie beispielsweise einem Sammelbehältnis, herzustellen.

Unter dem Begriff "pathologische Fluidansammlungen" sollen im Sinne der vorliegenden Erfindung krankhaftbedingte Ansammlungen von Körperflüssigkeiten und/oder Körpergasen verstanden werden. Als Körperflüssigkeiten seien beispielhaft Wundsekrete, Exsudate, Eiterflüssigkeiten und Darminhalte genannt.

Unter dem Ausdruck "Körperhöhle" soll im Sinne der vorliegenden Erfindung bevorzugt eine sogenannte Wundhöhle verstanden werden. Hierunter werden natürlich erworbene oder krankhaft bedingte Aussackungen oder Kanäle verstanden, die sich insbesondere mit Wundsekreten füllen können. Die krankhaft bedingten Aussackungen oder Kanäle sind dabei häufig die Folge von operativen Eingriffen, insbesondere von Anastomosen, wie beispielsweise Rektumanastomosen. Dort können sich im Ligatur- oder Nahtstellenbereich Aussackungen oder Kanäle bilden, die durch Infektionen schnell größer werden. Im Fall von Aussackungen im Bereich von Nahtstellen spricht man häufig auch von sogenannten Insuffizienzhöhlen. Krankhaft bedingte Aussackungen oder Kanäle können aber auch durch Infektionen und/oder Entzündungen, welche beispielsweise Fisteln bilden, verursacht werden.

Der Ausdruck "proximales Ende" meint im Sinne der vorliegenden Erfindung ein dem Rumpf eines Anwenders, in Regel Arztes, zugewandtes Ende, wohingegen der Ausdruck "distales Ende" ein vom Rumpf eines Anwenders, in der Regel Arztes, entferntes Ende definiert.

Wie bereits erwähnt, umfasst das Kit einen Fluidsammelkörper und einen Fluidkommunikationskörper sowie ferner eine Sicherungseinrichtung. Der Fluidsammelkörper und der Fluidkommunikationskörper sind derart ausgebildet, dass sie koaxial zueinander und verschieblich gegeneinander anordenbar sind, und der Fluidsammelkörper den Fluidkommunikationskörper teilweise, insbesondere nur teilweise, umgibt. Mit anderen Worten sind der Fluidsammelkörper und der Fluidkommunikationskörper derart koaxial zueinander und verschieblich gegeneinander anordenbar, dass der Fluidsammelkörper den Fluidkommunikationskörper teilweise, insbesondere nur teilweise, umgibt. Die Sicherungseinrichtung ist dazu ausgebildet, den Fluidsammelkörper gegen ein Durchschieben über ein distales Ende des Fluidkommunikationskörpers hinaus zu sichern. Wie bereits erwähnt, kann die Sicherungseinrichtung abhängig von ihrer Ausbildung obendrein zur Verankerung bzw. Befestigung und/oder Positionierung des Fluidsammelkörpers und/oder des Fluidkommunikationskörpers innerhalb einer Körperhöhle vorgesehen sein.

Vorzugsweise weisen der Fluidsammelkörper und der Fluidkommunikationskörper keine gegenseitigen Befestigungen oder Verbindungen auf.

Der Fluidkommunikationskörper weist die Sicherungseinrichtung auf.

Grundsätzlich kann die Sicherungseinrichtung einstückig bzw. monolithisch mit dem Fluidkommunikationskörper verbunden sein.

Alternativ hierzu kann es erfindungsgemäß jedoch ebenso vorgesehen sein, dass die Sicherungseinrichtung form-, kraft- und/oder stoffschlüssig mit dem Fluidkommunikationskörper verbunden ist. Beispielsweise kann die Sicherungseinrichtung mittels Klebung, Schweißung, Spritzguss oder dergleichen mit dem Fluidkommunikationskörper verbunden sein.

Erfindungsgemäß kann es insbesondere vorgesehen sein, dass die Sicherungseinrichtung derart ausgebildet ist, dass sie auf den Fluidkommunikationskörper aufgeschoben und insbesondere nach Aufschieben auf den Fluidkommunikationskörper arretiert werden kann.

Die Sicherungseinrichtung kann mit besonderem Vorteil derart angeordnet bzw. ausgebildet sein, dass auch während ihrer Positionierung, vorzugsweise zusammen mit dem Fluidkommunikationskörper, eine endoskopische Sichtkontrolle möglich ist.

Die Sicherungseinrichtung ist an einem distalen Ende des Fluidkommunikationskörpers angeordnet bzw. ausgebildet.

Die Sicherungseinrichtung kann in einer weiteren Ausführungsform komprimierbar ausgebildet sein oder in einem komprimierten Zustand vorliegen. Beispielsweise kann die Sicherungseinrichtung derart komprimierbar ausgebildet sein bzw. in einem derart komprimierten Zustand vorliegen, dass sie sich durch einen, sich vorzugsweise in Längsrichtung des Fluidkommunikationskörpers erstreckenden Kanal an das distale Ende des Fluidkommunikationskörpers befördern lässt.

In einer bevorzugten Ausführungsform ist die Sicherungseinrichtung entfaltbar bzw. expandierbar ausgebildet. Beispielsweise kann die Sicherungseinrichtung eine v-förmige Konfiguration mit komprimierbaren Schenkeln aufweisen. Alternativ kann die Sicherungseinrichtung ballonförmig ausgebildet sein, insbesondere in Form eines aufblasbaren Ballons vorliegen. Die in diesem Absatz beschriebenen Ausführungsformen haben insbesondere den Vorteil, dass die Sicherungseinrichtung nach ihrer Entfaltung bzw. Expansion dazu befähigt sein kann, den Fluidsammelkörper und/oder den Fluidkommunikationskörper innerhalb einer zu behandelnden Körperhöhle zu verankern bzw. zu befestigen und/oder den Fluidsammelkörper und/oder den Fluidkommunikationskörper in eine zu behandelnde Körperhöhle zu positionieren.

Die Sicherungseinrichtung weist, insbesondere in einem entfalteten oder expandierten Zustand, eine größere Querschnittsfläche, insbesondere einen größeren Außendurchmesser, auf als der Fluidkommunikationskörper oder gegebenenfalls der Fluidsammelkörper.

Die Sicherungseinrichtung besitzt in einer zweckmäßigen Ausführungsform eine atraumatische Form. Erfindungsgemäß kann es bevorzugt sein, dass die Sicherungseinrichtung kantenfrei oder rund, insbesondere wulst- oder kugelförmig, ausgebildet ist.

Alternativ kann die Sicherungseinrichtung platten-, scheiben- oder kegelförmig ausgebildet sein.

Zweckmäßigerweise weist die Sicherungseinrichtung ein bioverträgliches Material auf oder ist aus einem solchen Material gebildet. Geeignete Materialien können beispielsweise aus der Gruppe umfassend Polyethylen, Polypropylen, Polyvinylchlorid, Polyurethan, Silikon, Copolymere davon und Mischungen, insbesondere Blends, davon ausgewählt sein.

Das erfindungsgemäße Kit weist ein Führungselement auf. Unter einem Führungselement soll im Sinne der vorliegenden Erfindung ein Element verstanden werden, das eine geschiente bzw. geführte Positionierung des Fluidkommunikationskörpers und/oder Fluidsammelkörpers in eine Körperhöhle ermöglicht. Die Sicherungseinrichtung befindet sich auch in diesem Fall am distalen Ende des Fluidkommunikationskörpers bzw. ist dort angeordnet. Erfindungsgemäß handelt es sich bei dem Führungselement um einen Führungsdraht.

Der Fluidsammelkörper und der Fluidkommunikationskörper sind in einer weiteren Ausführungsform derart koaxial zueinander und verschieblich gegeneinander anordenbar, dass der Fluidkommunikationskörper distal und proximal aus dem Fluidsammelkörper austritt. Mit anderen Worten können der Fluidsammelkörper und der Fluidkommunikationskörper derart koaxial zueinander und verschieblich gegeneinander anordenbar sein, dass sich der Fluidsammelkörper zwischen den Enden des Fluidkommunikationskörpers befindet.

Der Fluidsammelkörper ist bevorzugt derart ausgebildet, dass er auf die Außenoberfläche des Fluidkommunikationskörpers aufgeschoben werden kann.

Der Fluidsammelkörper weist in einer weiteren Ausführungsform einen länglichen Hohlraum (längliches Lumen), insbesondere einen Kanal, auf, der sich vorzugsweise durchgehend in Längsrichtung des Fluidsammelkörpers erstreckt.

In einer weiteren Ausführungsform weist der Fluidkommunikationskörper einen länglichen Hohlraum (längliches Lumen), insbesondere einen Kanal, auf, der sich vorzugsweise durchgehend in Längsrichtung des Fluid-kommunikationskörpers erstreckt.

Der Hohlraum des Fluidsammelkörpers besitzt vorzugsweise einen Innendurchmesser, der wenigstens dem Außendurchmesser des Fluidkommunikationskörpers entspricht. Vorzugsweise ist der Innendurchmesser des Hohlraums des Fluidsammelkörpers (etwas) größer als der Außendurchmesser des Fluidkommunikationskörpers. Auf diese Weise ist ein einfaches Aufschieben des Fluidsammelkörpers auf den Fluidkommunikationskörper möglich.

Alternativ kann der Fluidsammelkörper anstelle des oben genannten Hohlraumes einen sich vorzugsweise in Längsrichtung des Fluidsammelkörpers erstreckenden Einschnitt, insbesondere Kreuzeinschnitt, aufweisen. Auch hierdurch ist ein Aufschieben des Fluidsammelkörpers auf den Fluidkommunikationskörper möglich.

Bei dem Fluidsammelkörper handelt es sich in einer weiteren Ausführungsform um eine Vielzahl von Fluidsammelkörpern, die sich insbesondere hinsichtlich Größe, Form, Härtegrad, Materialzusammensetzung und/oder Struktur, insbesondere Porosität, voneinander unterscheiden können. Hierdurch ist eine patientenspezifische Auswahl von geeigneten Fluidsammelkörpern bzw. Fluidsammelkörperqualitäten möglich. Die Fluidsammelkörper können insbesondere nach Art eines modularen Baukastensystems vorliegen.

Der Fluidsammelkörper weist in einer weiteren Ausführungsform eine Anschlusseinrichtung auf, die dazu ausgebildet ist, einen Anschluss des Fluidsammelkörpers an ein Schiebeinstrument (Pusher) zum Schieben des Fluidsammelkörpers in axialer Richtung des Fluidkommunikationskörpers zu bewirken. So lässt sich der Fluidsammelkörper sowohl in Richtung des distalen Endes als auch in Richtung des proximalen Endes des Fluidkommunikationskörpers verschieben.

Alternativ oder in Ergänzung hierzu kann der Fluidsammelkörper eine Anschlusseinrichtung aufweisen, die dazu ausgebildet ist, einen Anschluss des Fluidsammelkörpers an ein Zuginstrument (Drawer) zum Ziehen des Fluidsammelkörpers aus der Körperhöhle eines Patienten zu bewirken.

Die Anschlusseinrichtung ist vorzugsweise an einem proximalen Ende des Fluidsammelkörpers ausgebildet.

Bevorzugt ist die Anschlusseinrichtung des Fluidsammelkörpers komplementär zu einer weiteren, an einem Schiebe- oder Zuginstrument befindlichen Anschlusseinrichtung ausgebildet. Mit anderen Worten kann es erfindungsgemäß bevorzugt sein, wenn die Anschlusseinrichtung des Fluidsammelkörpers mit einer weiteren, an einem Schiebe- oder Zuginstrument befindlichen Anschlusseinrichtung verbunden bzw. konnektiert werden kann, um den Fluidsammelkörper in eine Körperhöhle zu schieben oder den Fluidsammelkörper aus einer Körperhöhle zu ziehen.

Um Gewebetraumatisierungen zu vermeiden, kann es weiterhin vorgesehen sein, dass die Anschlusseinrichtung innerhalb des Fluidsammelkörpers angeordnet ist. Mit anderen Worten kann es zum Zwecke der Vermeidung von Gewebetraumatisierungen von Vorteil sein, dass die Anschlusseinrichtung nicht aus dem Fluidsammelkörper herausragt. Beispielsweise kann sich die Anschlusseinrichtung 1 mm bis 20 mm, bevorzugt 5 mm bis 15 mm, unterhalb der Außenoberfläche des Fluidsammelkörpers befinden.

Die Verbindung der Anschlusseinrichtung mit dem Fluidsammelkörper, einem Schiebe- und/oder Zuginstrument kann auf einer lösbaren oder nichtlösbaren Verbindung beruhen.

Vorzugsweise ist die Anschlusseinrichtung als Konnektor, insbesondere ausgewählt aus der Gruppe umfassend Schnapp-Konnektor, Bajonett-Konnektor, Luer-Lock-Konnektor und Schraub-Konnektor, ausgebildet.

In einer weiteren Ausführungsform umfasst das Kit als weitere Kitkomponente einen Temperatursensor. Der Temperatursensor kann beispielsweise in dem Fluidkommunikationskörper eingebaut sein. Vorzugsweise ist der Temperatursensor zur Erfassung von Infektionen, insbesondere in einer Körperhöhle eines Patienten, vorgesehen.

Das Kit umfasst in einer weiteren Ausführungsform einen Katheter, insbesondere einen Katheter, der an einem seiner Enden, vorzugsweise an einem distalen Ende, einen Ballon, vorzugsweise einen aufblasbaren Ballon, aufweist (Fogarty-Katheter).

In einer weiteren Ausführungsform umfasst das Kit als weitere Kitkomponente ein Endoskop. Das Endoskop weist vorzugsweise Halteeinrichtungen zum Platzieren des Fluidkommunikationskörpers in eine Körperhöhle eines Patienten auf.

Die Halteeinrichtungen können beispielsweise auf das Endoskop aufgeklipst oder aufgeschoben sein.

Insbesondere können die Halteeinrichtungen derart ausgebildet sein, dass durch ein nachfolgendes Aufschieben des Fluidsammelkörpers auf den Fluidkommunikationskörper Letzterer, vorzugsweise zusammen mit der Sicherungseinrichtung, vom Endoskop bzw. den Halteinrichtungen abgelöst wird.

In einer weiteren Ausführungsform weist das Kit als weitere Kitkomponente ein Fixierelement, insbesondere ein Zusammendrückelement, auf. Das Fixier- bzw. Zusammendrückelement ist mit besonderem Vorteil derart ausgebildet, dass es eine Fixierung des Fluidsammelkörpers, gegebenenfalls unter teilweisem Zusammendrücken desselben, auf dem Fluidkommunikationskörper bewirkt, ohne jedoch hierdurch die gegenseitige Verschieblichkeit von Fluidsammelkörper und Fluidkommunika-tionskörper nennenswert zu beeinträchtigen.

Das Fixier- bzw. Zusammendrückelement hat insbesondere die Funktion, den Fluidsammelkörper zusätzlich gegen ein ungewolltes Durchschieben über das distale Ende des Fluidkommunikationskörpers hinaus abzusichern.

Bevorzugt ist das Fixier- bzw. Zusammendrückelement ausgewählt aus der Gruppe umfassend einen Faden, insbesondere in Form einer Fadenschlaufe, eine Klemme bzw. ein Clip, einen Ring und einen Behälter, insbesondere in Form eines Rohres oder Schlauches.

Der Fluidsammelkörper besitzt in einer zweckmäßigen Ausführungsform eine offenporöse Struktur.

Der Fluidsammelkörper kann Poren mit einem Durchmesser von 100 µm bis 1500 µm, insbesondere 200 µm bis 1000 µm, vorzugsweise 400 µm bis 800 µm, aufweisen.

Der Fluidsammelkörper ist in bevorzugten Ausführungsformen zylindrisch ausgebildet. Beispielsweise kann der Fluidsammelkörper als Zylinder mit einem kreisförmigen oder nichtkreisförmigen Querschnitt, insbesondere ovalen, dreieckigen, quadratischen, trapezoidalen, rhomboiden, pentagonalen oder hexagonalen Querschnitt, ausgebildet sein. Bevorzugt ist eine kreiszylindrische oder im Wesentlichen kreiszylindrische Ausbildung des Fluidsammelkörpers.

Der Fluidsammelkörper kann des Weiteren ein Flockmaterial, vorzugsweise in Form von Fasern (Flockfasern), aufweisen bzw. mit einem solchen Material versehen sein. Bevorzugt ist das Flockmaterial auf der Außenoberfläche des Fluidsammelkörpers angeordnet bzw. ausgebildet.

Das Flockmaterial selbst kann grundsätzlich resorbierbar, teilresorbierbar oder nicht resorbierbar sein. In anderen Worten kann das Flockmaterial ein resorbierbares, teilresorbierbares oder nicht resorbierbares Material, bevorzugt Polymer, insbesondere Copolymer, aufweisen bzw. aus einem solchen Material, bevorzugt Polymer, insbesondere Copolymer, gebildet sein.

So kann das Flockmaterial grundsätzlich ein synthetisches bzw. technisches Polymer und/oder ein Biopolymer, d.h. ein natürlich vorkommendes Polymer, aufweisen bzw. aus solchen Polymeren gebildet sein.

Bevorzugt weist das Flockmaterial ein Polymer auf bzw. ist aus einem Polymer gebildet, welches ausgewählt ist aus der Gruppe umfassend Polyolefine, Polyamide, Polyester, Polyurethane, insbesondere thermoplastische Polyurethane, Polyhydroxyalkanoate, Polysaccharide, technische Polysaccharide, oxidierte und nicht oxidierte Polysaccharide, Aminogruppen tragende Polysaccharide, Aldehydgruppen tragende Polysaccharide, Mucopolysaccharide, Proteine Strukturproteine, extrazelluläre Proteine, Faserproteine, globuläre Proteine, Enzyme, Antikörper, Blutgerinnungsfaktoren, Copolymere davon, Salze davon, Stereoisomere davon und Mischungen davon.

Beispielsweise kann das Flockmaterial ausgewählt sein aus der Gruppe umfassend Polyethylen, Polyethylen mit niedriger Dichte, Polyethylen mit hoher Dichte, hochmolekulares Polyethylen, ultrahochmolekulares Polyethlyen, Polypropylen, Polyethylenterephthalat, Polypropylenterephthalat, Polybutylenterephthalat, Polyacrylnitril, Polyamid 6, Polyamid 6-6, Polyamid 6-12, Polyamid 12, Seide, insbesondere Kunst- oder Spinnenseide, Polytetrafluorethylen, Polyvinylidendifluorid, Polytetrafluorpropylen, Polyhexafluorpropylen, Polyvinylalkohol, Polyglykolid bzw. Polyglykolsäure, Polylactid bzw. Polymilchsäure, Polydioxanon, Poly-3-hydroxybutyrat bzw. Poly-3-hydroxybuttersäure, Poly-4-hydroxybutyrat bzw. Poly-4-hydroxybuttersäure, Polytrimethylencarbonat, Poly-ε-Caprolacton, Baumwolle, Cellulose, Cellulosederivate wie beispielsweise Alkylcellulosen, Methylcellulose, Hydroxyalkylcellulosen, Hydroxymethylcellulose, Hydroxyethylcellulose, Hydroxypropylcellulose, Carboxyalkylcellulosen, Carboxymethylcellulose, Stärke, Amylose, Amylopektin, Dextran, Dextrin, Chitin, Chitosan, Hyaluronsäure, Dextransulfat, Heparin, Heparansulfat, Chondroitinsulfat, Dermatansulfat, Kollagen, Gelatine, Elastin, Retikulin, Fibronektin, Laminin, Fibrin, Fibrinogen, Albumin, Copolymere davon, Salze davon, Stereoisomere davon und Mischungen davon.

Ein mögliches Copolymer für das Flockmaterial stellt beispielsweise ein Copolymer aus Glykolid und Lactid, insbesondere in einem Gewichtsverhältnis von 9:1 bis 1:9, insbesondere 7:3 bis 3:7, dar.

Ein mögliches Terpolymer, insbesondere Triblockterpolymer, für das Flockmaterial stellt zum Beispiel ein Terpolymer, insbesondere Triblockterpolymer, aus Glykolid, Trimethylencarbonat und ε-Caprolacton dar. Ein derartiges Terpolymer ist beispielsweise unter der Bezeichnung Monosyn® kommerziell erhältlich.

Das Flockmaterial weist in einer weiteren Ausführungsform ein in wässrigen Flüssigkeiten, insbesondere wässrigen Lösungen, wässrigen Suspensionen, wässrigen Pufferlösungen, wässrigen Elektrolytlösungen und/oder Körperflüssigkeiten, quellbares oder lösliches Material auf bzw. ist in einer weiteren Ausführungsform aus einem solchen Material gebildet. Ein geeignetes Material ist vorzugsweise ausgewählt aus der Gruppe umfassend Polyvinylpyrrolidon, Polyvinylalkohol, Polyethylenglykol bzw. Polyethylenglykol basierte Copolymere, Viskose, Baumwolle, Cellulose, Cellulosederivate wie beispielsweise Alkylcellulosen, Methylcellulose, Hydroxyalkylcellulosen, Hydroxymethylcellulose, Hydroxyethylcellulose, Hydroxypropylcellulose, Carboxyalkylcellulosen, Carboxymethylcellulose, Stärke, Amylose, Amylopektin, Dextran, Dextrin, Chitin, Chitosan, Hyaluronsäure, Dextransulfat, Heparin, Heparansulfat, Chondroitinsulfat, Dermatansulfat, Kollagen, Gelatine, Elastin, Retikulin, Fibronektin, Laminin, Fibrin, Fibrinogen, Albumin, Copolymere davon, Salze davon, Stereoisomere davon und Mischungen davon.

Ein wasserlösliches oder in Wasser quellbares Flockmaterial, insbesondere wie im vorangegangenen Absatz beschrieben, eignet sich insbesondere für eine Additivierung mit pharmazeutischen bzw. medizinischen Wirkstoffen, auf welche im Folgenden noch näher eingegangen werden wird. Solche Wirkstoffe können sodann mit besonderem Vorteil während einer Therapie freigesetzt werden. Auf diese Weise kann das erfindungsgemäße Kit als Wirkstofffreisetzungsvehikel (drug delivery device) verwendet werden.

In einer weiteren Ausführungsform weist das Flockmaterial ein physiologisch oder biologisch aktives Material auf bzw. ist aus einem solchen Material gebildet. Unter dem Ausdruck "physiologisch aktives Material" bzw. "biologisch aktives Material" soll ein Material verstanden werden, welches unter kosmetischen und/oder medizinischen Gesichtspunkten vorteilhafte Wirkungen im Körper eines Patienten bewirken kann.

Insbesondere kann das Flockmaterial einen kosmetischen Wirkstoff, biologischen Wirkstoff, pharmazeutischen bzw. medizinischen Wirkstoff oder Mischungen davon aufweisen bzw. aus einem solchen Wirkstoff oder einer solchen Wirkstoffmischung gebildet sein.

Zum Beispiel kann das Flockmaterial einen Wirkstoff aufweisen bzw. aus einem Wirkstoff gebildet sein, welcher ausgewählt ist aus der Gruppe umfassend antimikrobielle, insbesondere antibiotische, Wirkstoffe, wundheilungsfördernde Wirkstoffe, desinfizierende Wirkstoffe, hautpflegende Wirkstoffe, onkologische Wirkstoffe, Narben pflegende bzw. Narben verhindernde Wirkstoffe, entzündungshemmende Wirkstoffe, schmerzlindernde Wirkstoffe, blutgerinnungsfördernde Wirkstoffe, Wachstumsfaktoren, zelldifferenzierende Faktoren, zelladhäsive Faktoren, zellrekrutierende Faktoren, Zellrezeptoren, zellbindende Faktoren, Zytokine, Peptide, Strukturproteine, extrazelluläre Proteine wie beispielsweise Kollagen, Polysaccharide wie beispielsweise Hyaluronsäure, Oligonukleotide, Polynukleotide, DNA, RNA, Salze davon, Stereoisomere davon und Mischungen davon.

Insbesondere kann das Flockmaterial einen Wirkstoff aufweisen bzw. aus einem Wirkstoff gebildet sein, welcher ausgewählt ist aus der Gruppe umfassend FGF (Fibroblast Growth Factor), TGF (Transforming Growth Factor), PDGF (Platelet-Derived Growth Factor), EGF (Epidermal Growth Factor), GMCSF (Granulocyte-Macrophage Colony Stimulation Factor), VEGF (Vascular Endothelial Growth Factor), IGF (Insulin-like Growth Factor), HGF (Hepatocyte Growth Factor), IL-1B (Interleukin-1-B), IL-8 (Interleukin-8), NGF (Nerve Growth Factor), BMPs bzw. knochenmorphogenetische Proteine wie beispielsweise BMP-1 (knochenmorphogenetisches Protein 1) und/oder BMP-2 (knochenmorphogenetisches Protein 2), Salze davon, Stereoisomere davon und Mischungen davon.

Als geeignete antimikrobielle Wirkstoffe seien beispielhaft Wirkstoffe genannt, welche aus der Gruppe ausgewählt sind, umfassend Biguanide, Polyhexamethylenbiguanid (PHMB), Triclosan, Chlorhexidin, Gentamycin, Kupfer, Zink, Silber, Gold, Salze davon, Stereoisomere davon und Mischungen davon.

Es versteht sich, dass das Flockmaterial auch eine Mischung der in den vorangegangenen Ausführungsformen beschriebenen Materialien aufweisen oder aus einer solchen Mischung bestehen kann.

Vorzugsweise ist das Flockmaterial mit dem Fluidsammelkörper bzw. dessen Oberfläche, insbesondere Außenoberfläche, über eine die Oberfläche des Fluidsammelkörpers wenigstens teilweise, insbesondere vollständig, bedeckende Klebstoffschicht verbunden.

Grundsätzlich kann die Klebstoffschicht resorbierbar, teilresorbierbar oder nicht resorbierbar ausgebildet sein. In anderen Worten kann die Klebstoffschicht ein resorbierbares, teilresorbierbares oder nicht resorbierbares Material, bevorzugt Polymer, insbesondere Copolymer, aufweisen bzw. aus einem solchen Material, bevorzugt Polymer, insbesondere Copolymer, gebildet sein.

Für die Klebstoffschicht kommen grundsätzlich alle Materialien in Betracht, die sich für die Durchführung einer Beflockung eignen, d.h. für eine Verbindung des Flockmaterials mit der Klebstoffschicht und damit mit der Oberfläche des Fluidsammelkörpers.

So kann die Klebstoffschicht grundsätzlich ein synthetisches bzw. technisches Polymer und/oder ein Biopolymer, d.h. ein natürlich vorkommenden Polymer, aufweisen bzw. aus solchen Polymeren gebildet sein.

Beispielsweise kann die Klebstoffschicht ein nicht resorbierbares Material, insbesondere nicht resorbierbares Polymer, aufweisen bzw. aus einem solchen Material, insbesondere Polymer, gebildet sein, welches vorzugsweise ausgewählt ist aus der Gruppe umfassend Schmelzklebstoffe oder Heißklebstoffe, Polyvinylalkohol, Polyvinylacetat, Polyvinylchlorid, Polyvinylpropionat, Polyacrylate, Cyanoacrylate, Poly-α-Olefine, Polyurethane, thermoplastische Polyurethane, thermoplastische Polyurethan-Elastomere, Vinylacetatcopolymere, insbesondere modifizierte Vinylacetatcopolymere, Polyester, Vinylpyrrolidon-Vinylacetat-Copolymer, Polycarbonate, Gummi, Polyoxymethylen, Acrylnitril-Butadien-Styrol-Copolymer, Polyethylenglykol basierte Hydrogele, Harze wie beispielsweise synthetische Harze, Wachse wie beispielsweise synthetische Wachse und/oder Bienenwachse und Mischungen davon.

Geeignete thermoplastische Polyurethan-Elastomere sind zum Beispiel unter den Bezeichnungen Texin® oder Desmopan® kommerziell erhältlich.

Bevorzugt weist die Klebstoffschicht einen Schmelz- oder Heißklebstoff auf bzw. ist aus einem solchen Klebstoff gebildet. Geeignete Schmelz- oder Heißklebstoffe können ausgewählt sein aus der Gruppe umfassend Schmelzklebstoffe auf Basis von Polyamiden, auf Basis von Polyester-Elastomeren, auf Basis von Polyolefinen, insbesondere Polyethylen, auf Basis von amorphen Poly-α-Olefinen, auf Basis von Polybuten-1, auf Basis von Ethylen-Vinylacetat-Copolymer, auf Basis von Polyurethan-Elastomeren, auf Basis von Copolyamid-Elastomeren, auf Basis von Vinylpyrrolidon-Vinylacetat-Copolymeren und Mischungen davon.

Als geeignete Harze seien beispielhaft Harze genannt, welche aus der Gruppe umfassend Urethan-Harze, Epoxid-Harze, Polyurethan-Harze, Melanin-Harze, Phenol-Harze, PolyesterHarze, Polyamid-Harze, Vinylester-Harze und Mischungen davon ausgewählt sind.

Alternativ oder in Kombination zu den in den vorangegangenen Ausführungsformen beschriebenen nicht resorbierbaren Materialien, insbesondere Polymeren, kann die Klebstoffschicht ein resorbierbares Material, insbesondere Polymer, aufweisen bzw. aus einem solchen Material, insbesondere Polymer, gebildet sein, welches vorzugsweise ausgewählt ist aus der Gruppe umfassend Polyhydroxyalkanoate, Polyglykolid bzw. Polyglykolsäure, Polylactid bzw. Polymilchsäure, Polydioxanon, Poly-3-hydroxybutyrat bzw. Poly-3-hydroxybuttersäure, Poly-4-hydroxybutyrat bzw. Poly-4-hydroxybuttersäure, technische Polysaccharide, oxidierte oder nicht oxidierte Polysaccharide, Aminogruppen tragende Polysaccharide, Aldehydgruppen tragende Polysaccharide, Dextranaldehyd, Cellulose, Cellulosederivate wie beispielsweise Alkylcellulosen, Methylcellulose, Hydroxyalkylcellulosen, Hydroxymethylcellulose, Hydroxy-ethylcellulose, Hydroxypropylcellulose, Carboxyalkylcellulosen, Carboxymethylcellulose, Copolymere davon, Salze davon, Stereoisomere davon und Mischungen davon.

Alternativ oder in Kombination zu den in den vorangegangenen Ausführungsformen genannten Materialien, insbesondere Polymeren, für die Klebstoffschicht kann diese in einer weiteren Ausführungsform ein Biopolymer aufweisen bzw. aus einem Biopolymer gebildet sein, welches vorzugsweise ausgewählt ist aus der Gruppe umfassend Polysaccharide, Mucopolysaccharide, Stärke, Amylose, Amylopektin, Dextran, Dextrin, Cellulose, Chitin, Chitosan, Hyaluronsäure, Dextransulfat, Heparin, Heparansulfat, Chondroitinsulfat, Dermatansulfat, Strukturproteine, extrazelluläre Proteine, faserförmige Proteine, globuläre Proteine, Kollagen, Gelatine, Elastin, Retikulin, Fibronektin, Laminin, Fibrin, Albumin, Copolymere davon, Salze davon, Stereoisomere davon und Mischungen davon.

Es versteht sich, dass die Klebstoffschicht auch eine Mischung der in den vorangegangenen Ausführungsformen beschriebenen Materialien aufweisen oder aus einer solchen Mischung bestehen kann.

In einer weiteren Ausführungsform kann die Klebstoffschicht aus einem Material, insbesondere einem Polymer wie beispielsweise einem thermoplastischen Polymer, gebildet sein, welches eine niedrigere Schmelztemperatur besitzt als ein Material, insbesondere Polymer wie beispielsweise thermoplastisches Polymer, aus dem der Fluidsammelkörper gebildet ist. Dadurch ist mit besonderem Vorteil eine spezifische thermische Aktivierung der Klebstoffschicht erzielbar, ohne dass die Integrität der Fluidsammelkörperstruktur beeinträchtigt wird. Insbesondere kann die Schmelztemperatur des zur Herstellung der Klebstoffschicht verwendeten Materials niedriger sein als die Schmelztemperatur des Flockmaterials. Geeignete Materialien bzw. Polymere für die in diesem Absatz beschriebenen Ausführungsformen stellen neben Polyethylen und/oder Polypropylen grundsätzlich alle industriellen Schmelzkleber bzw. Schmelzklebermischungen dar. Insoweit wird daher auch ausdrücklich auf die vorangegangene Beschreibung Bezug genommen.

In einer alternativen Ausführungsform kann das Flockmaterial auch unmittelbar, d.h. ohne eine Klebstoffschicht, mit dem Fluidsammelköper bzw. dessen Oberfläche, insbesondere Außenoberfläche, verbunden sein.

Der Fluidsammelkörper kann insbesondere in Form eines Rohres oder Schlauches ausgebildet sein. Bevorzugt entspricht der Innendurchmesser des Rohres bzw. Schlauches dem Außendurchmesser bzw. im Wesentlichen dem Außendurchmesser des Fluidkommunikationskörpers. Auf diese Weise kann gewährleistet werden, dass der rohr- bzw. schlauchförmige Fluidsammelkörper - bei Anlegen eines Unterdrucks oder Vakuums an das proximale Ende des Fluidkommunikationskörpers - möglichst dicht an dem Fluidkommunikationskörper anliegt. Das Rohr bzw. der Schlauch weist vorzugsweise ein Flockmaterial, insbesondere in Form von Flockfasern, auf. Das Flockmaterial kann über eine Klebstoffschicht oder unmittelbar, d.h. ohne eine Klebstoffschicht, mit der Oberfläche, insbesondere Außenoberfläche, des Rohres bzw. Schlauches verbunden sein. Das Flockmaterial bewirkt mit besonderem Vorteil eine Aufnahme, insbesondere ein Aufsaugen, von pathologischen Fluidansammlungen. Weiterhin ist es bevorzugt, wenn das Rohr bzw. der Schlauch an seinem distalen Ende Öffnungen aufweist, um eine Fluidkommunikation mit dem Fluidkommunikationskörper zu erlauben. Bezüglich geeigneter Flock- und Klebstoffmaterialien wird auf die in den vorherigen Ausführungsformen beschriebenen Materialien Bezug genommen.

Alternativ kann der Fluidsammelkörper als Textilstruktur, insbesondere als dreidimensionale Textilstruktur, vorliegen. Beispielsweise kann der Fluidsammelkörper als Abstandsgewirk, Abstandsgeflecht, Rundgeflecht oder Rundgestrick, insbesondere mit einer Velourstruktur, vorliegen.

Alternativ kann der Fluidsammelkörper als Ring, insbesondere ringkreisförmige Scheibe, ausgebildet sein.

Erfindungsgemäß ist es indes besonders bevorzugt, wenn der Fluidsammelkörper in Form eines Schwammes oder Schaumes, bevorzugt eines offenporösen Schwammes oder Schaumes, ausgebildet ist.

Weiterhin kann es vorgesehen sein, dass der Fluidsammelkörper in Einzelsegmente, insbesondere in schwamm- oder schaumförmige Einzelsegmente, untergliedert ist.

Der Fluidsammelkörper kann in weiteren Ausführungsformen Additive, insbesondere ausgewählt aus der Gruppe umfassend antimikrobielle, insbesondere antibiotische, Wirkstoffe, antiseptische Wirkstoffe, desinfizierende Wirkstoffe, entzündungshemmende Wirkstoffe, schmerzlindernde Wirkstoffe, geruchshemmende Wirkstoffe und Mischungen davon, aufweisen. Im Übrigen wird auf die im Zusammenhang des Flockmaterials genannten Wirkstoffe Bezug genommen.

Grundsätzlich kann der Fluidsammelkörper resorbierbar, teilresorbierbar oder nicht resorbierbar ausgebildet sein.

So kann der Fluidsammelkörper ein Polymer aufweisen bzw. aus einem Polymer gebildet sein, wobei das Polymer vorzugsweise ausgewählt ist aus der Gruppe umfassend Polyolefine, Polyethylen, Polypropylen, Polyvinylchlorid, Polyethylenterephthalat, Polypropylenterephthalat, Poly-butylenterephthalat, Polyurethan, Polyvinylalkohol, Polyester, Copolymere davon und Mischungen, insbesondere Blends, davon.

Vorzugsweise weist der Fluidsammelkörper Polyurethan auf bzw. ist aus Polyurethan gebildet. Das Polyurethan kann ein aliphatisches Polyurethan sein und insbesondere linear, verzweigt oder cyclisch sein. Weiterhin kann das Polyurethan resorbierbar oder nicht resorbierbar sein.

Ein lineares aliphatisches Polyurethan ist erfindungsgemäß bevorzugt.

Das Polyurethan kann weiterhin aus makromolekularen und/oder niedermolekularen aliphatischen Diolen und aliphatischen Diisocyanaten gebildet sein. Geeignete makromolekulare Diole stellen Polycarbonate, wie beispielsweise 1,6-Hexandiol-polycarbonat, dar. Geeignete niedermolekulare Diole können aus der Gruppe umfassend 2,2,4-Trimethylhexandiol, 2,4,4-Trimethylhexandiol, 1,4-Butandiol und Mischungen davon ausgewählt sein. Als aliphatische Diisocyanate kommen vorzugsweise cycloaliphatische Diisocyanate, insbesondere 4,4-Dicyclohexylmethan-4,4'-diisocyanat oder 1,4-Cyclohexyldiisocyanat, in Frage. Des Weiteren kann das Polyurethan aus verschiedenen Diolen und/oder Diisocyanaten hergestellt sein.

Das Polyurethan ist in einer weiteren Ausführungsform ausgewählt aus der Gruppe umfassend aliphatisches Polycarbonaturethan, Silikon-Polycarbonaturethan, Polyetherurethan, Silikon-Polyetherurethan, Polyurethanether und Mischungen, insbesondere Blends, davon.

In einer alternativen oder ergänzenden Ausführungsform weist der Fluidsammelkörper ein resorbierbares Polymer auf oder ist aus einem solchen Polymer gebildet, wobei das Polymer vorzugsweise ausgewählt ist aus der Gruppe umfassend Polyglykolid, Polylactid, Poly-ε-caprolacton, Trimethylencarbonat, Poly-para-dioxanon, Poly-3-hydroxybutyrat, Poly-4-hydroxybutyrat, Copolymere davon, Salze davon, Stereoisomere davon und Mischungen, insbesondere Blends, davon.

In einer weiteren Ausführungsform liegt der Fluidsammelkörper mittels eines Materials, wie beispielsweise Polyvinylalkohol (PVA), komprimiert vor. Eine Entfaltung bzw. Expansion des Fluidsammelkörpers nach dessen Positionierung in eine Körperhöhle ist sodann durch einfaches Anspülen, beispielsweise mit einer physiologisch verträglichen Pufferlösung, möglich.

Der Fluidkommunikationskörper ist bevorzugt schlauchförmig, insbesondere in Form wenigstens eines Schlauches, vorzugsweise wenigstens eines Drainageschlauches, ausgebildet.

Besonders bevorzugt liegt der Fluidkommunikationskörper in Form eines Schlauches, insbesondere eines Drainageschlauches, vor.

In einer alternativen Ausführungsform handelt es sich bei dem Fluidkommunikationskörper um mehrere Schläuche. Beispielsweise kann der Fluidkommunikationskörper in Form von zwei Schläuchen vorliegen, wobei ein Schlauch vorzugsweise zur Drainage bzw. Ableitung der Fluidansammlungen und der andere Schlauch vorzugsweise zur Spülung des Fluidsammelkörpers vorgesehen sind. Bezüglich geeigneter Spülflüssigkeiten wird auf die im Folgenden noch genannten Spülflüssigkeiten Bezug genommen.

Alternativ kann der Fluidkommunikationskörper mehrlumig bzw. mehrkanalig ausgebildet sein. Zu diesem Zweck kann ein originärer Hohlraum (Lumen), insbesondere ein originärer Kanal, des Fluidkommunikationskörpers mittels einer oder gegebenenfalls mehrerer Trennwände in zwei oder mehr Hohlräume, insbesondere Kanäle, untergliedert sein.

Erfindungsgemäß kann es weiterhin vorgesehen sein, dass der Fluidkommunikationskörper sowohl zur Drainage als auch zur Spülung vorgesehen ist. Zur Spülung kann der Fluidkommunikationskörper mit einer Spülflüssigkeit beschickt werden, welche beispielsweise ausgewählt ist aus der Gruppe umfassend Kochsalzlösung, Pufferlösung, antimikrobiell wirksame Lösung, entzündungshemmende Lösung, geruchshemmende Lösung und Mischungen davon.

Der Fluidkommunikationskörper ist zweckmäßigerweise aus einem fluiddichten, insbesondere flüssigkeitsdichten, Material gebildet. Beispielsweise kann der Fluidkommunikationskörper aus Polyethylen, Polypropylen, Polyvinylchlorid, Polyurethan oder Mischungen, insbesondere Blends, davon gebildet sein.

Um insbesondere eine fluidleitende Verbindung zwischen dem Fluidsammelkörper und dem Fluidkommunikationskörper zu gewährleisen, ist es in einer weiteren Ausführungsform vorgesehen, dass der Fluidkommunikationskörper Öffnungen, insbesondere Löcher, Poren, Perforationen oder dergleichen, und/oder Einschnitte, aufweist.

In einer zweckdienlichen Ausführungsform befinden sich die Öffnungen und/oder Einschnitte in einem Bereich des Fluidkommunikationskörpers, der von dem Fluidsammelkörper umgeben, insbesondere bedeckt, ist. Vorzugsweise befinden sich die Öffnungen am distalen Ende des Fluidkommunikationskörpers. Damit lässt sich mit besonderem Vorteil ein gleichmäßiger Unterdruck an dem Fluidsammelkörper anlegen. Zudem ermöglichen die Öffnungen bzw. Einschnitte eine schnellere und damit effizientere Ableitung der von dem Fluidsammelkörper aufgenommenen Fluide. Die Öffnungen bzw. Einschnitte können dabei lediglich im Bereich des distalen Endes des Fluidkommunikationskörpers ausgebildet sein.

Im Falle eines rohr- bzw. schlauchförmigen und insbesondere beflockten Fluidsammelkörpers kann es zudem zweckmäßig sein, wenn auch dieser Öffnungen, insbesondere Löcher, Poren, Perforationen oder dergleichen, und/oder Einschnitte an seinem distalen Ende aufweist. Auf diese Weise ist eine Fluidleitung zwischen dem Fluidsammelkörper und dem Fluidkommunikationskörper möglich.

In einer weiteren Ausführungsform kann der Fluidkommunikationskörper mit seinem proximalen Ende an eine Saug- oder Vakuumquelle, insbesondere Saug- oder Vakuumpumpe, angeschlossen werden. Die Saug- bzw. Vakuumquelle erzeugt üblicherweise einen Unterdruck oder Sog von 25 mmHg bis 600 mmHg, insbesondere 25 mmHg bis 200 mmHg, bevorzugt 50 mmHg bis 150 mmHg, weiter bevorzugt 80 mmHg bis 125 mmHg. Dadurch sind eine besonders rasche Säuberung von insbesondere infektiösen Körperhöhlen sowie eine nachfolgend rasche Bildung von Granulationsgewebe erzielbar. Mit besonderem Vorteil kann der Behandlungserfolg mittels einer Feinjustierung des Unterdrucks bzw. Sogs gezielt beeinflusst werden.

Bei der Saug- bzw. Vakuumquelle kann es sich mit besonderem Vorteil um eine tragbare Saug- bzw. Vakuumpumpe handeln. Dadurch lässt sich die Mobilität des Patienten während einer Behandlung aufrechterhalten.

Die abgeleiteten Fluidmengen werden normalerweise in hierfür vorgesehene Fluidauffangkörper, insbesondere Sammelbehältnisse, beispielsweise Kanister oder Vakuumflaschen, aufgefangen. Den Fluidauffangkörpern sind in der Regel eine Saug- oder Vakuumquelle, insbesondere der oben beschriebenen Art, vorgeschaltet und stehen mit dieser zweckmäßigerweise über geeignete Verbindungsschläuche in Kontakt. Um die Saug- bzw. Vakuumquelle nicht zu kontaminieren, kann zwischen den Fluidauffangkörpern und der Saug- bzw. Vakuumquelle ein steriler Filter vorgesehen sein.

In einer weiteren Ausführungsform kann wenigstens ein Ende des Fluidkommunikationskörpers verschließbar ausgebildet sein. Erfindungsgemäß kann es insbesondere vorgesehen sein, dass beide Enden des Fluidkommuniktionskörpers verschließbar ausgebildet sind. Abhängig vom Bedarf kann der Fluidkommunikationskörper an einem Ende geöffnet werden, um einen zur Ableitung von pathologischen Fluidansammlungen erforderlichen Unterdruck anlegen zu können.

Gemäß einem zweiten Aspekt wird ein medizinisches Produkt zur Ableitung von pathologischen Fluidansammlungen, insbesondere zur Durchführung einer endoluminalen und/oder -kavitären Vakuumtherapie, offenbart.

Das Produkt umfasst einen Fluidsammelkörper und einen Fluidkommunikationskörper.

Der Fluidsammelkörper und der Fluidkommunikationskörper sind koaxial zueinander und verschieblich, insbesondere ohne Befestigungen bzw. Verbindungen, gegeneinander angeordnet, wobei der Fluidsammelkörper den Fluidkommunikationskörper teilweise, insbesondere nur teilweise, umgibt.

Das Produkt umfasst ferner eine Sicherungseinrichtung. Die Sicherungseinrichtung ist dazu ausgebildet, den Fluidsammelkörper gegen ein Durchschieben über ein distales Ende des Fluidkommunikationskörpers hinaus zu sichern. Abhängig von der Ausgestaltung der Sicherungseinrichtung kann diese obendrein zur Verankerung bzw. Befestigung und/oder Positionierung des Fluidsammelkörpers und/oder des Fluidkommunikationskörpers innerhalb einer Körperhöhle vorgesehen sein und insoweit zusätzlich als Verankerungs- bzw. Befestigungs- und/oder Positionierungseinrichtung wirken.

Der Fluidsammelkörper und der Fluidkommunikationskörper sind in einer weiteren Ausführungsform derart koaxial zueinander und verschieblich gegeneinander angeordnet, dass der Fluidkommunikationskörper distal und proximal aus dem Fluidsammelkörper austritt. Mit anderen Worten ist der Fluidsammelkörper in dieser Ausführungsform zwischen den Enden des Fluidkommunikationskörpers angeordnet. Dabei kann eine mittige Anordnung des Fluidsammelkörpers zwischen den Enden des Fluidkommunikationskörpers bevorzugt sein.

In einer weiteren Ausführungsform ist der Fluidsammelkörper auf den Fluidkommunikationskörper aufgeschoben.

Bezüglich weiterer Merkmale und Vorteile des Produkts, insbesondere des Fluidsammelkörpers, des Fluidkommunikationskörpers sowie der Sicherungseinrichtung, wird vollständig auf die im Rahmen des ersten Erfindungsaspektes gemachten Ausführungen Bezug genommen.

Gemäß einem dritten Aspekt wird ein Fluidkommunikationskörper offenbart, der vorzugsweise an seinem distalen Ende eine Sicherungseinrichtung aufweist.

Die Sicherungseinrichtung ist dazu ausgebildet, einen Fluidsammelkörper, der koaxial zu dem Fluidkommunikationskörper und verschieblich gegen diesen anordenbar ist, wobei der Fluidsammelkörper den Fluidkommunikationskörper teilweise, insbesondere nur teilweise, umgibt, gegen ein Durchschieben über ein distales Ende des Fluidkommunikationskörpers hinaus zu sichern. Abhängig von der Ausgestaltung der Sicherungseinrichtung kann diese obendrein zur Verankerung bzw. Befestigung und/oder Positionierung eines Fluidsammelkörpers und/oder des Fluidkommunikationskörpers innerhalb einer Körperhöhle vorgesehen sein und insoweit zusätzlich als Verankerungs- bzw. Befestigungs- und/oder Positionierungseinrichtung wirken.

Bezüglich weiterer Merkmale und Vorteile des im Rahmen des dritten Aspektes abgehandelten Fluidkommunikationskörpers, insbesondere der in diesem Zusammenhang erwähnten Komponenten Fluidsammelkörper und Sicherungseinrichtung, wird ebenfalls auf die im Rahmen des ersten Erfindungsaspektes gemachten Ausführungen Bezug genommen.

Gemäß einem vierten Aspekt wird ein Führungselement offenbart, umfassend eine Sicherungseinrichtung, die dazu ausgebildet ist, einen Fluidsammelkörper, der koaxial zu einem Fluidkommunikationskörper und verschieblich gegen diesen anordenbar ist, wobei der Fluidsammelkörper den Fluidkommunikationskörper teilweise, insbesondere nur teilweise, umgibt, gegen ein Durchschieben über ein distales Ende des Fluidkommunikationskörpers hinaus zu sichern. Abhängig von der Ausgestaltung der Sicherungseinrichtung kann diese obendrein zur Verankerung bzw. Befestigung und/oder Positionierung eines Fluidsammelkörpers und/oder eines Fluidkommunikationskörpers innerhalb einer Körperhöhle vorgesehen sein und insoweit zusätzlich als Verankerungs- bzw. Befestigungs- und/oder Positionierungseinrichtung wirken.

Das Führungselement ist vorzugsweise als Führungsdraht ausgebildet.

Bezüglich weiterer Merkmale und Vorteile des im Rahmen des vierten Erfindungsaspektes abgehandelten Führungselementes, insbesondere der in diesem Zusammenhang erwähnten Komponenten Fluidsammelkörper, Fluidkommunikationskörper sowie Sicherungseinrichtung, wird ebenso vollständig auf die im Rahmen des ersten Erfindungsaspektes gemachten Ausführungen Bezug genommen.

Weitere Merkmale und Vorteile der Erfindung ergeben sich aus der nachfolgenden Beschreibung bevorzugter Ausführungsformen in Form von Figuren, Figurenbeschreibungen sowie der Unteransprüche. Dabei können einzelne Merkmale jeweils für sich alleine oder zu mehreren in Kombination miteinander verwirklicht sein. Die beschriebenen Ausführungsformen dienen lediglich der Erläuterung der vorliegenden Erfindung, die in keiner Weise darauf beschränkt sein soll.

### Figurenbeschreibung

Die Figuren zeigen schematisch:
- Fig. 1:: eine Ausführungsform eines nicht erfindungsgemäßen Kits,
- Fig. 2:: eine Ausführungsform eines nicht erfindungsgemäßen Produkts,
- Fig. 3:: eine weitere Ausführungsform eines nicht erfindungsgemäßen Kits,
- Fig. 4:: eine Ausführungsform eines erfindungsgemäßen Kits,
- Fig. 5:: eine weitere Ausführungsform eines nicht erfindungsgemäßen Kits,
- Fig. 6:: ein Zuginstrument zum Austausch eines Fluidsammelkörpers,
- Fig. 7:: eine weitere Ausführungsform eines nicht erfindungsgemäßen Kits und
- Fig. 8:: eine weitere Ausführungsform eines nicht erfindungsgemäßen Kits.

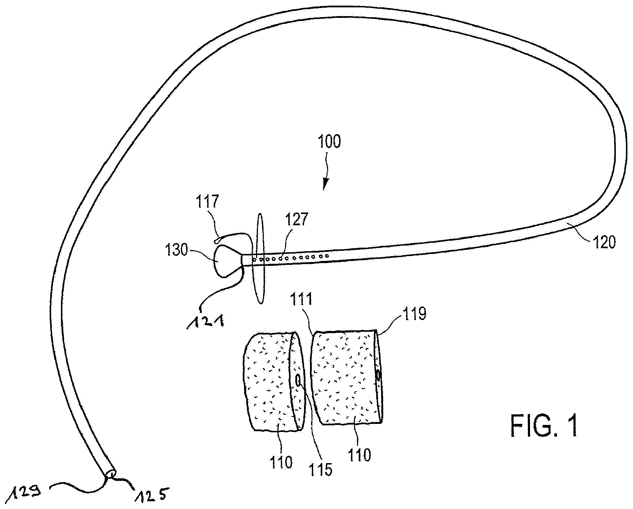

Fig. 1 zeigt schematisch ein nicht erfindungsgemäßes medizinisches Kit 100. Das Kit 100 ist zur Ableitung von pathologischen Fluidansammlungen, wie beispielsweise Wundsekretansammlungen aus menschlichen oder tierischen Körperhöhlen, vorgesehen. Vorzugsweise eignet sich das medizinische Kit 100 zur Durchführung einer endoluminalen und/oder -kavitären Vakuumtherapie.

Das Kit 100 weist einen oder - wie dargestellt - mehrere, beispielsweise zwei, Fluidsammelkörper 110, einen Fluidkommunikationskörper 120 sowie eine Sicherungseinrichtung 130 auf.

Der (bzw. die) Fluidsammelkörper 110, der Fluidkommunikationskörper 120 sowie ggf. die Sicherungseinrichtung 130 können räumlich voneinander getrennt vorliegen.

Der (bzw. die) Fluidsammelkörper 110 und der Fluidkommunikationskörper 120 sind derart ausgebildet, dass sie koaxial zueinander und verschieblich gegeneinander anordenbar sind, wobei der (bzw. die) Fluidsammelkörper 110 den Fluidkommunikationskörper 120 teilweise umgibt.

Dies kann beispielsweise dadurch realisiert werden, dass der (bzw. die) Fluidsammelkörper 110 einen sich in Längsrichtung des Fluidsammelkörpers 110 (bzw. der Fluidsammelkörper 110) erstreckenden Kanal 115 und der Fluidkommunikationskörper 120 einen sich in Längsrichtung des Fluidkommunikationskörpers 120 erstreckenden Kanal 125 aufweist, wobei der Innendurchmesser des Kanals 115 dem Außendurchmesser des Fluidkommunikationskörpers 120 entspricht oder größer ist als der Außendurchmesser des Fluidkommunikationskörpers 120.

Der (bzw. die) Fluidsammelkörper 110 besitzt ein distales Ende 111 und ein proximales Ende 119. Entsprechend besitzt der Fluidkommunikationskörper 120 ein distales Ende 121 und ein proximales Ende 129.

Die Sicherungseinrichtung 130 befindet sich an dem distalen Ende 121 des Fluidkommunikationskörpers 120. Die Sicherungseinrichtung 130 kann, wie in Fig. 1 dargestellt, wulstförmig ausgebildet sein. Aufgrund ihrer wulstförmigen Ausbildung besitzt die Sicherungseinrichtung 130 eine größere Querschnittsfläche als der Fluidkommunikationskörper 120 und verhindert auf diese Weise ein ungewolltes Durchschieben des Fluidsammelkörpers 110 (bzw. der Fluidsammelkörper 110) über das distale Ende 121 des Fluidkommunikationskörpers 120 hinaus.

Grundsätzlich kann die Sicherungseinrichtung 130 einstückig bzw. monolithisch mit dem distalen Ende 121 des Fluidkommunikationskörpers 120 verbunden sein.

Alternativ kann die Sicherungseinrichtung 130 lösbar mit dem distalen Ende 121 des Fluidkommunikationskörpers 120 verbunden sein. Beispielsweise kann die Sicherungseinrichtung 130 auf das distale Ende 121 des Fluidkommunikationskörpers 120 aufsteckbar ausgebildet sein.

Der (bzw. die) Fluidsammelkörper 110 kann weiterhin, wie dargestellt, in Form eines Kreiszylinders ausgebildet sein.

Vorzugsweise ist der (bzw. die) Fluidsammelkörper 110 als offenporöser Schaum, insbesondere offenporöser Polyurethanschaum, ausgebildet.

Um eine zusätzliche Absicherung gegen ein unbeabsichtigtes Durchschieben des Fluidsammelkörpers 110 (bzw. der Fluidsammelkörper 110) über das distale Ende 121 des Fluidkommunikationskörpers 120 hinaus bereitzustellen, kann es erfindungsgemäß vorgesehen sein, dass das Kit ferner ein Fixierelement 117, beispielsweise in Form einer Fadenschlaufe, aufweist. Mittels des Fixierelementes 117 kann der Fluidsammelkörper 110 am distalen Ende 111 etwas zusammengedrückt, beispielsweise eingeschnürt, und insbesondere stabilisiert werden, ohne hierdurch jedoch die gegenseitige Verschieblichkeit/Verschiebbarkeit von Fluidsammelkörper 110 und Fluidkommunikationskörper 120 nennenswert zu beeinträchtigen.

Um eine Kommunikation von Fluiden, insbesondere Wundsekreten, zwischen dem Fluidsammelkörper 110 (bzw. den Fluidsammelkörpern 110) und dem Fluidkommunikationskörper 120 zu ermöglichen, weist Letzterer im Bereich seines distalen Endes 121 Öffnungen 127 auf. Über die Öffnungen 127 können die von dem Fluidsammelkörper 110 (bzw. den Fluidsammelkörpern 110) aufgenommenen Fluide, insbesondere bei Anlegen eines Unterdrucks oder Vakuums, abgeleitet werden.

Der Fluidkommunikationskörper 120 ist vorzugsweise schlauchförmig ausgebildet und zweckmäßigerweise aus einem fluiddichten, insbesondere flüssigkeitsdichten, Material, wie beispielsweise Polyethylen, Poly-propylen, Polyvinylchlorid und/oder Polyurethan gebildet.

Zur endgültigen Abführung von pathologischen Fluidansammlungen aus einer menschlichen oder tierischen Körperhöhle kann der Fluidkommunikationskörper 120 an seinem proximalen Ende 129 mit einer Unterdruck- oder Vakuumquelle verbunden werden.

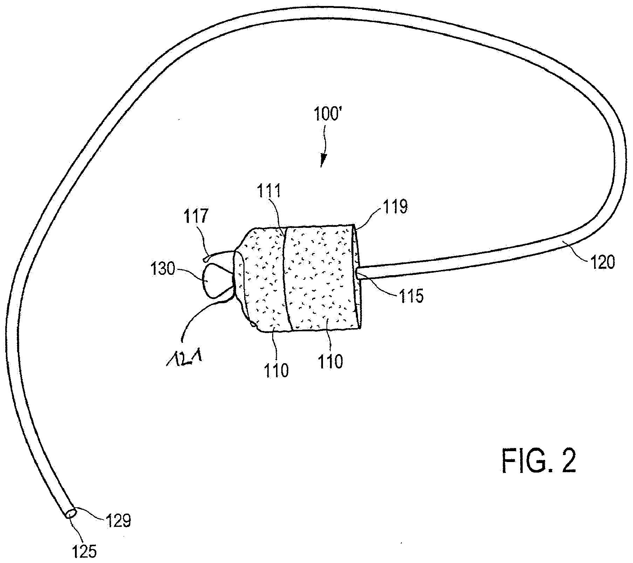

Fig. 2 zeigt schematisch ein nicht erfindungsgemäßes medizinisches Produkt 100'. Das Produkt 100' ist vorzugsweise zur Ableitung von pathologischen Fluidansammlungen, insbesondere zur Durchführung einer endoluminalen und/oder -kavitären Vakuumtherapie, vorgesehen.

Das Produkt 100' umfasst einen oder - wie dargestellt - mehrere, beispielsweise zwei, Fluidsammelkörper 110, einen Fluidkommunikationskörper 120 sowie eine Sicherungseinrichtung 130.

Der (bzw. die) Fluidsammelkörper 110 und der Fluidkommunikationskörper 120 sind koaxial zueinander und verschieblich gegeneinander angeordnet, wobei der (bzw. die) Fluidsammelkörper 110 den Fluidkommunikationskörper 120 teilweise umgibt (umgeben).

Das Produkt 100' lässt sich in besonders vorteilhafter Weise mittels des in Fig. 1 schematisch dargestellten Kits durch einfaches Aufschieben des Fluidsammelkörpers 110 (bzw. der Fluidsammelkörper 110) auf den Fluidkommunikationskörper 120 herstellen. Hierzu weist der (bzw. die) Fluidsammelkörper 110 einen Kanal 115 auf, dessen Innendurchmesser dem Außendurchmesser des Fluidkommunikationskörpers 120 entspricht, vorzugsweise größer ist als der Außendurchmesser des Fluidkommunikationskörpers 120.

Bezüglich weiterer Merkmale und Vorteile des Produkts 100', insbesondere des Fluidsammelkörpers 110 (bzw. der Fluidsammelkörper 110), des Fluidkommunikationskörpers 120 sowie der Sicherungseinrichtung 130, wird vollständig auf die Beschreibung zur Figur 1 Bezug genommen.

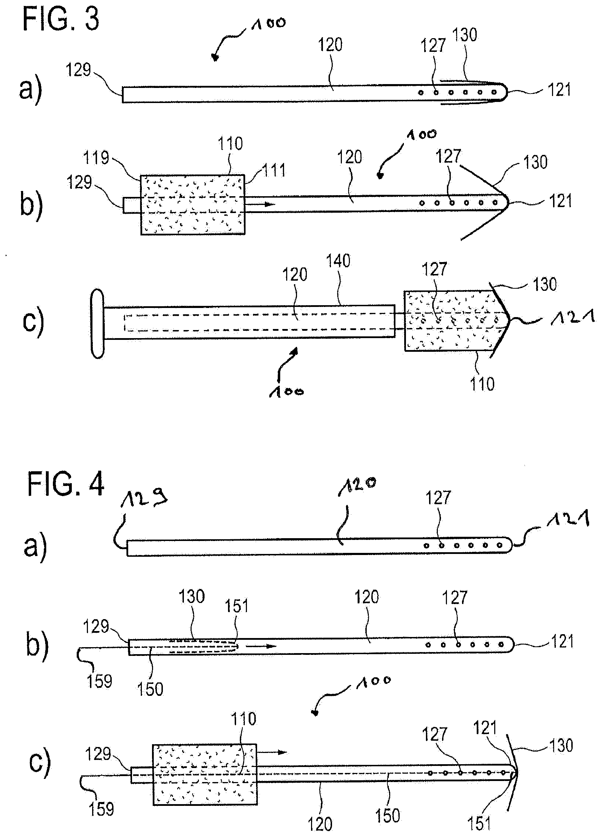

Fig. 3a zeigt schematisch eine weitere Variante eines nicht erfindungsgemäßen Kits 100. Das Kit 100 umfasst einen Fluidkommunikationskörper 120 sowie eine Sicherungseinrichtung 130, die an dem distalen Ende 121 des Fluidkommunikationskörpers 120 angeordnet ist.

Im Bereich des distalen Endes 121 befinden sich Öffnungen 127, die dazu vorgesehen sind, eine fluidleitende Verbindung mit einem Fluidsammelkörper 110 herzustellen.

Die Sicherungseinrichtung 130 kann, wie dargestellt, in Form einer am distalen Ende 121 anliegenden Platte angeordnet sein, welche sich mittels eines auf den Fluidkommunikationskörpers 120 aufgeschobenen Fluidsammelkörpers 110 (siehe Figur 3b), insbesondere unter Zuhilfenahme eines Schiebeinstruments 140 (siehe Figur 3c), entfalten, insbesondere aufklappen, lässt.

Bezüglich weiterer Merkmale und Vorteile des Kits 100, insbesondere des Fluidsammelkörpers 110, des Fluidkommunikationskörpers 120 sowie der Sicherungseinrichtung 130, wird vollständig auf die Beschreibung zur Figur 1 Bezug genommen.

Fig. 4a zeigt schematisch einen Fluidkommunikationskörper 120 mit einem proximalen Ende 129 und einem distalen Ende 121 sowie mit Öffnungen 127. Der Fluidkommunikationskörper 120 besitzt einen sich in dessen Längsrichtung durchgehend erstreckenden Kanal.

Figur 4b zeigt ein Führungselement 150 mit einem distalen Ende 151 und einem proximalen Ende 159. Am distalen Ende 151 ist eine komprimierbare, insbesondere zusammenklappbare, Sicherungseinrichtung 130 angeordnet, wodurch die Sicherungseinrichtung 130 durch den oben genannten Kanal des Fluidkommunikationskörpers 120 in Richtung auf dessen distales Ende 121 geschoben werden kann.

Sobald die Sicherungseinrichtung 130 aus dem distalen Ende 121 des Fluidkommunikationskörpers 120 heraustritt, entfaltet sich die Sicherungseinrichtung 130. Die Sicherungseinrichtung 130 besitzt im entfalteten, insbesondere ausgeklappten, Zustand eine Querschnittsfläche, die größer ist als die Querschnittsfläche des Führungselementes 150 sowie des Fluidkommunikationskörpers 120. Hierdurch wird verhindert, dass ein im Anschluss daran auf den Fluidkommunikationskörper 120 aufgeschobener Fluidsammelkörper 110 über das distale Ende 121 hinaus geschoben wird (siehe Figur 4c).

Bezüglich weiterer Merkmale und Vorteile, insbesondere des Fluidsammelkörpers 110, des Fluidkommunikationskörpers 120 sowie der Sicherungseinrichtung 130, wird vollständig auf die Beschreibung zur Figur 1 Bezug genommen.

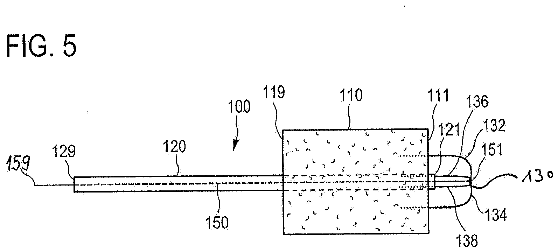

Fig. 5 zeigt schematisch eine Variante eines nicht erfindungsgemäßen Kits 100. Das Kit 100 umfasst einen Fluidsammelkörper 110, einen Fluidkommunikationskörper 120 sowie ein

Führungselement 150. Das Führungselement 150 besitzt an seinem distalen Ende 151 eine vierarmig ausgestaltete Sicherungseinrichtung 130. Die Arme 132; 134; 136; 138 der Sicherungseinrichtung 130 sind zweckmäßigerweise derart voneinander beabstandet, dass sie den Fluidsammelkörper 110 umgreifen können, sobald dieser das distale Ende 121 des Fluidkommunikationskörpers 120 erreicht hat. Auf diese Weise wird ein unbeabsichtigtes Durchschieben des Fluidsammelkörpers 110 über das distale Ende 121 hinaus verhindert, ohne dass hierdurch die gegenseitige Verschieblichkeit/Verschiebbarkeit von Fluidsammelkörper 110 und Fluidkommunikationskörper 120 signifikant gestört wird.

Bezüglich weiterer Merkmale und Vorteile des Kits 100, insbesondere des Fluidsammelkörpers 110, des Fluidkommunikationskörpers 120, der Sicherungseinrichtung 130 sowie des Führungselementes 150, wird vollständig auf die Beschreibung zur Figur 1 Bezug genommen.

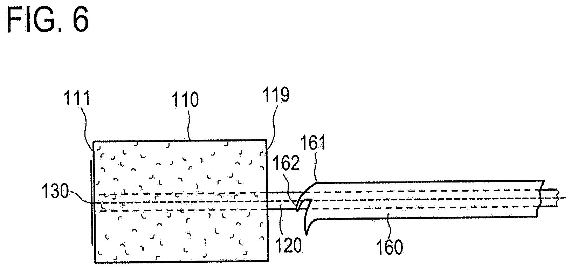

Fig. 6 zeigt schematisch ein Zuginstrument 160, welches an seinem distalen Ende 161 Greifelemente 162 aufweist, mittels derer ein positionierter Fluidsammelkörper 110, beispielsweise durch einfaches Eindrehen des Zuginstruments 160 in den Fluidsammelkörper 110, insbesondere im Bereich von dessen proximalen Ende 119, aus einer menschlichen oder tierischen Körperhöhle entfernt werden kann.

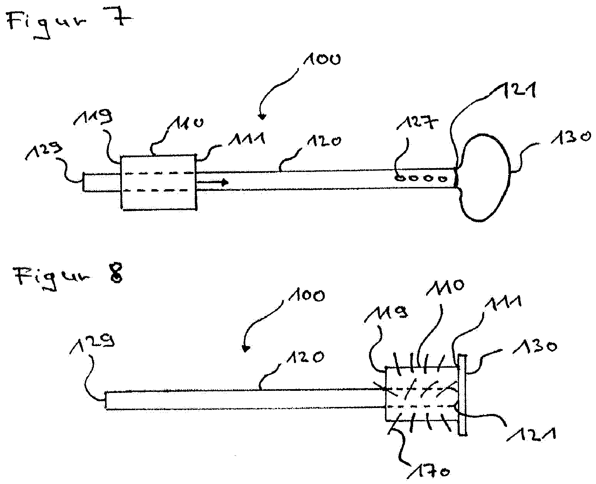

Figur 7 zeigt schematisch eine weitere Variante eines nicht erfindungsgemäßen Kits 100.

Das Kit 100 umfasst einen Fluidsammelkörper 110 sowie einen Fluidkommunikationskörper 120.

An dem distalen Ende 121 des Fluidkommunikationskörpers 120 ist eine Sicherungseinrichtung 130 in Form eines aufblasbaren Ballons angeordnet. Der Ballon 130 kann beispielsweise Bestandteil eines Fogarty-Katheters sein.

Wird der Fluidsammelkörper 110 in Längsrichtung des Fluidkommunikationskörpers 120 geschoben, so verhindert die ballonförmige Sicherungseinrichtung 130, dass der Fluidsammelkörper 110 über das distale Ende 121 hinaus geschoben wird. Obendrein eignet sich die ballonförmige Sicherungseinrichtung 130 für eine Positionierung und insbesondere Verankerung sowohl des Fluidsammelkörpers 110 als auch des Fluidkommunikationskörpers 120 innerhalb einer menschlichen oder tierischen Körperhöhle.

Bezüglich weiterer Merkmale und Vorteile des Kits 100, insbesondere des Fluidsammelkörpers 110, des Fluidkommunikationskörpers 120 sowie der Sicherungseinrichtung 130, wird vollständig auf die Beschreibung zur Figur 1 Bezug genommen.

Figur 8 zeigt eine weitere Variante eines nicht erfindungsgemäßen Kits 100. Das Kit 100 umfasst ebenfalls einen Fluidsammelkörper 110 sowie einen Fluidkommunikationskörper 120.

An dem distalen Ende 121 des Fluidkommunikationskörpers 120 befindet sich eine Sicherungseinrichtung 130, welche - wie dargestellt - eine plattenförmige Ausgestaltung besitzen kann.

Der Fluidsammelkörper 110 ist bei dieser Kitvariante bevorzugt als Rohr oder Schlauch ausgebildet. Zur Aufnahme von pathologischen Fluiden, wie beispielsweise Wundsekreten oder Exsudaten, ist der rohr- bzw. schlauchförmige Fluidsammelkörper 110 an seiner Außenoberfläche mit einem vorzugsweise faserförmigen Flockmaterial 170 versehen. Das Flockmaterial 170 kann dabei über eine Klebstoffschicht (nicht dargestellt) oder unmittelbar mit der Außenoberfläche des Fluidsammelkörpers 110 verbunden sein.

Der rohr- bzw. schlauchförmige Fluidsammelkörper 110 besitzt bevorzugt einen Innendurchmesser, der (im Wesentlichen) dem Außendurchmesser des Fluidkommunikationskörpers 120 entspricht, damit der Fluidsammelkörper 110 den Fluidkommunikationskörper 120 möglichst passgenau umgeben kann. Alternativ kann der Fluidsammelkörper 110 aus einem Material gebildet sein, welches sich bei Anlegen eines Unterdrucks oder Vakuums möglichst passgenau um den Fluidkommunikationskörper 120 anlegt.

Bezüglich weiterer Merkmale und Vorteile des Kits 100, insbesondere des Fluidsammelkörpers 110, des Fluidkommunikationskörpers 120 sowie der Sicherungseinrichtung 130, wird vollständig auf die Beschreibung zur Figur 1 Bezug genommen.

## Patentansprüche

1. Medizinisches Kit (100) zur Ableitung von pathologischen Fluidansammlungen, insbesondere zur Durchführung einer endoluminalen und/oder -kavitären Vakuumtherapie, umfassend einen Fluidsammelkörper (110) und einen Fluidkommunikationskörper (120), wobei der Fluidsammelkörper (110) und der Fluidkommunikationskörper (120) derart koaxial zueinander und verschieblich gegeneinander anordenbar sind, dass der Fluidsammelkörper (110) den Fluidkommunikationskörper (120) teilweise umgibt, wobei das Kit (100) ferner eine Sicherungseinrichtung (130) aufweist, die dazu ausgebildet ist, den Fluidsammelkörper(110) gegen ein Durchschieben über ein distales Ende (121) des Fluidkommunikationskörpers (120) hinaus zu sichern, wobei die Sicherungseinrichtung (130) eine Querschnittsfläche aufweist, die größer ist als die Querschnittsfläche des Fluidkommunikationskörpers (120), und am distalen Ende (121) des Fluidkommunikationskörpers (120) ausgebildet ist, **dadurch gekennzeichnet, dass** das Kit (100) als weitere Kitkomponente einen Führungsdraht (150) umfasst.

2. Medizinisches Kit (100) nach Anspruch 1, **dadurch gekennzeichnet, dass** die Sicherungseinrichtung (130) komprimierbar ausgebildet ist oder in einem komprimierten Zustand vorliegt.

3. Medizinisches Kit (100) nach Anspruch 1 oder 2, **dadurch gekennzeichnet, dass** die Sicherungseinrichtung (130) in einem entfalteten oder expandierten Zustand eine Querschnittsfläche aufweist die größer ist als die Querschnittfläche des Fluidkommunikationskörpers (120).

4. Medizinisches Kit (100) nach einem der vorhergehenden Ansprüche, **dadurch gekennzeichnet, dass** die Sicherungseinrichtung (130) ballonförmig ausgebildet ist, vorzugweise in Form eines aufblasbaren Ballons vorliegt.

5. Medizinisches Kit (100) nach einem der Ansprüche 1 bis 3, **dadurch gekennzeichnet, dass** die Sicherungseinrichtung (130) platten- oder scheibenförmig ausgebildet ist.

6. Medizinisches Kit (100) nach einem der vorhergehenden Ansprüche, **dadurch gekennzeichnet, dass** sowohl der Fluidsammelkörper (110) als auch der Fluidkommunikationskörper (120) jeweils einen länglichen Hohlraum, insbesondere Kanal (115; 125), aufweisen, der sich durchgehend in Längsrichtung des Fluidsammelkörpers (110) bzw. Fluidkommunikationskörpers (120) erstreckt, wobei der Innendurchmesser des Hohlraums des Fluidsammelkörpers (110) dem Außendurchmesser des Fluidkommunikationskörpers (120) entspricht, vorzugsweise größer ist als der Außendurchmesser des Fluidkommunikationskörpers (120).

7. Medizinisches Kit (100) nach einem der vorhergehenden Ansprüche, **dadurch gekennzeichnet, dass** es sich bei dem Fluidsammelkörper (110) um eine Vielzahl von Fluidsammelkörpern (110), insbesondere in unterschiedlichen Größen, Formen, Materialzusammensetzungen und/oder Strukturen, handelt.

8. Medizinisches Kit (100) nach einem der vorhergehenden Ansprüche, **dadurch gekennzeichnet, dass** der Fluidsammelkörper (110) eine Anschlusseinrichtung aufweist, die dazu ausgebildet ist, einen Anschluss des Fluidsammelkörpers (110) an ein Schiebeinstrument (140) zum Schieben des Fluidsammelkörpers (110) in axialer Richtung des Fluidkommunikationskörpers (120) und/oder an ein Zuginstrument (160) zum Ziehen des Fluidsammelkörpers (110) aus einer Körperhöhle eines Patienten zu bewirken.

9. Medizinisches Kit (100) nach einem der vorhergehenden Ansprüche, **dadurch gekennzeichnet, dass** das Kit (100) als weitere Kitkomponente einen Temperatursensor, vorzugsweise zur Erfassung von Infektionen in einer Körperhöhle eines Patienten, umfasst.

10. Medizinisches Kit (100) nach einem der vorhergehenden Ansprüche, **dadurch gekennzeichnet, dass** das Kit (100) als weitere Kitkomponente ein Endoskop umfasst, wobei das Endoskop vorzugsweise Halteeinrichtungen zum Platzieren des Fluidkommunikationskörpers in eine Körperhöhle eines Patienten aufweist.

11. Medizinisches Kit (100) nach einem der vorhergehenden Ansprüche, **dadurch gekennzeichnet, dass** das Kit (100) als weitere Kitkomponente ein Fixierelement (117), insbesondere ein Zusammendrückelement, umfasst, welches vorzugsweise ausgewählt ist aus der Gruppe umfassend einen Faden, insbesondere in Form einer Fadenschlaufe, einen Ring und eine Klemme.

12. Medizinisches Kit (100) nach einem der vorhergehenden Ansprüche, **dadurch gekennzeichnet, dass** der Fluidsammelkörper (110) in Form eines offenporösen Schaumes und der Fluidkommunikationskörper (120) in Form wenigstens eines Schlauches ausgebildet ist.

## Claims

1. Medical kit (100) for draining pathological fluid accumulations, in particular for carrying out an endoluminal and/or endocavitary vacuum therapy, comprising a fluid collecting body (110) and a fluid communication body (120), wherein the fluid collecting body (110) and the fluid communication body (120) are arrangeable coaxial to each other and displaceable in relation to each other such that the fluid collecting body (110) partially surrounds the fluid communication body (120), wherein the kit (100) further includes a securing device (130) which is configured to secure the fluid collecting body (110) against pushing through beyond a distal end (121) of the fluid communication body (120), wherein the securing device (130) has a cross-sectional area that is greater than the cross-sectional area of the fluid communication body (120) and is provided at the distal end (121) of the fluid communication body (120),
**characterized in that**
the kit (100) comprises a guide wire (150) as a further kit component.

2. Medical kit (100) according to claim 1, **characterized in that** the securing device (130) is designed to be compressible or is provided in a compressed condition.

3. Medical kit (100) according to claim 1 or 2, **characterized in that** the securing device (130) in an unfolded or expanded condition has a cross-sectional area that is greater than the cross-sectional area of the fluid communication body (120).

4. Medical kit (100) according to any of the preceding claims, **characterized in that** the securing device (130) has a balloon shape, preferably is provided in the form of an inflatable balloon.

5. Medical kit (100) according to any of the claims 1 to 3, **characterized in that** the securing device (130) has a plate or disc shape.

6. Medical kit (100) according to any of the preceding claims, **characterized in that** both the fluid collecting body (110) and the fluid communication body (120) each include an elongate cavity, in particular a channel (115; 125), which extends continuously in the longitudinal direction of the fluid collecting body (110) or fluid communication body (120), wherein the inner diameter of the cavity of the fluid collecting body (110) corresponds to the outer diameter of the fluid communication body (120), preferably is greater than the outer diameter of the fluid communication body (120).

7. Medical kit (100) according to any of the preceding claims, **characterized in that** the fluid collecting body (110) includes a plurality of fluid collecting bodies (110), in particular in different sizes, shapes, material compositions and/or structures.

8. Medical kit (100) according to any of the preceding claims, **characterized in that** the fluid collecting body (110) has a connector device which is configured to effect connection of the fluid collecting body (110) to a sliding instrument (140) for sliding the fluid collecting body (110) in the axial direction of the fluid communication body (120) and/or to a pull instrument (160) for pulling the fluid collecting body (110) from a body cavity of a patient.

9. Medical kit (100) according to any of the preceding claims, **characterized in that** the kit (100) comprises a temperature sensor as a further kit component, preferably for detecting infections in a body cavity of a patient.

10. Medical kit (100) according to any of the preceding claims, **characterized in that** the kit (100) comprises an endoscope as a further kit component, wherein the endoscope preferably has retaining devices for placing the fluid communication body in a body cavity of a patient.

11. Medical kit (100) according to any of the preceding claims, **characterized in that** the kit (100) comprises a fixing element (117) as a further kit component, in particular a compression element, which preferably is selected from the group comprising a string, in particular in the form of a string loop, a ring and a clamp.

12. Medical kit (100) according to any of the preceding claims, **characterized in that** the fluid collecting body (110) is configured in the form of an open-porous foam and the fluid communication body (120) is configured in the form of at least one tube.

## Revendications

1. Kit médical (100) pour drainer des fluides pathologiques recueillis, en particulier pour effectuer une thérapie sous vide endoluminale et/ou endocavitaire, comprenant un corps de collecte de fluide (110) et un corps de communication de fluide (120), le corps de collecte de fluide (110) et le corps de communication de fluide (120) pouvant être disposés coaxialement l'un à l'autre et de manière déplaçable l'un par rapport à l'autre de telle sorte que le corps de collecte de fluide (110) entoure en partie le corps de communication de fluide (120), le kit (100) présentant en outre un dispositif de fixation (130) qui est réalisé pour protéger le corps de collecte de fluide (110) contre une poussée au-delà d'une extrémité distale (121) du corps de communication de fluide (120), le dispositif de fixation (130) présentant une surface en section transversale qui est supérieure à la surface en section transversale du corps de communication de fluide (120) et qui est réalisée à l'extrémité distale (121) du corps de communication de fluide (120), **caractérisé en ce que** le kit (100) comprend, en tant que composant de kit supplémentaire, un fil-guide (150).

2. Kit médical (100) selon la revendication 1, **caractérisé en ce que** le dispositif de fixation (130) est réalisé de manière compressible ou se présente dans un état comprimé.

3. Kit médical (100) selon la revendication 1 ou 2, **caractérisé en ce que** le dispositif de fixation (130), dans un état déplié ou expansé, présente une surface en section transversale qui est supérieure à la surface en section transversale du corps de communication de fluide (120).

4. Kit médical (100) selon l'une quelconque des revendications précédentes, **caractérisé en ce que** le dispositif de fixation (130) est réalisé sous forme de ballonnet, de préférence sous la forme d'un ballonnet gonflable.

5. Kit médical (100) selon l'une quelconque des revendications 1 à 3, **caractérisé en ce que** le dispositif de fixation (130) est réalisé sous forme de plaque ou de disque.

6. Kit médical (100) selon l'une quelconque des revendications précédentes, **caractérisé en ce que** le corps de collecte de fluide (110) ainsi que le corps de communication de fluide (120) présentent chacun une cavité allongée, en particulier un canal (115 ; 125), qui s'étend de manière continue dans la direction longitudinale du corps de collecte de fluide (110) ou du corps de communication de fluide (120), le diamètre intérieur de la cavité du corps de collecte de fluide (110) correspondant au diamètre extérieur du corps de communication de fluide (120), de préférence étant supérieur au diamètre extérieur du corps de communication de fluide (120).

7. Kit médical (100) selon l'une quelconque des revendications précédentes, **caractérisé en ce que** le corps de collecte de fluide (110) est constitué par une pluralité de corps de collecte de fluide (110), en particulier de tailles, formes et compositions de matériaux et/ou de structures différentes.

8. Kit médical (100) selon l'une quelconque des revendications précédentes, **caractérisé en ce que** le corps de collecte de fluide (110) présente un dispositif de raccordement qui est réalisé de manière à provoquer un raccordement du corps de collecte de fluide (110) à un instrument de poussée (140) pour pousser le corps de collecte de fluide (110) dans la direction axiale du corps de communication de fluide (120) et/ou à un instrument de traction (160) pour tirer le corps de collecte de fluide (110) hors d'une cavité corporelle d'un patient.

9. Kit médical (100) selon l'une quelconque des revendications précédentes, **caractérisé en ce que** le kit (100) comprend, en tant que composant de kit supplémentaire, un capteur de température, de préférence pour détecter des infections dans une cavité corporelle d'un patient.

10. Kit médical (100) selon l'une quelconque des revendications précédentes, **caractérisé en ce que** le kit (100) comprend, en tant que composant de kit supplémentaire, un endoscope, l'endoscope présentant de préférence des dispositifs de retenue pour placer le corps de communication de fluide dans une cavité corporelle d'un patient.

11. Kit médical (100) selon l'une quelconque des revendications précédentes, **caractérisé en ce que** le kit (100), en tant que composant de kit supplémentaire, comprend un élément de fixation (117), en particulier un élément de compression qui est de préférence choisi dans le groupe comprenant un fil, en particulier sous forme de boucle de fil, une bague et une pince.

12. Kit médical (100) selon l'une quelconque des revendications précédentes, **caractérisé en ce que** le corps de collecte de fluide (110) est réalisé sous la forme d'une mousse à pores ouverts et le corps de communication de fluide (120) est réalisé sous la forme d'au moins un tuyau.
